(19) Europäisches Patentamt
European Patent Office
Office européen des brevets

(11) Veröffentlichungsnummer : **0 372 281 B1**

(12) **EUROPÄISCHE PATENTSCHRIFT**

(45) Veröffentlichungstag der Patentschrift : **14.06.95 Patentblatt 95/24**

(51) Int. Cl.[6] : **G03C 7/388,** G03C 7/392, G03C 7/32, G03C 7/305

(21) Anmeldenummer : **89121374.6**

(22) Anmeldetag : **18.11.89**

# (54) Fotografisches Aufzeichnungsmaterial.

(30) Priorität : **02.12.88 DE 3840619**

(43) Veröffentlichungstag der Anmeldung : **13.06.90 Patentblatt 90/24**

(45) Bekanntmachung des Hinweises auf die Patenterteilung : **14.06.95 Patentblatt 95/24**

(84) Benannte Vertragsstaaten : **BE DE FR GB**

(56) Entgegenhaltungen :
**DE-A- 2 936 842**
**FR-A- 2 078 921**
**JP-A- 6 285 242**
**JP-B- 4 916 058**
**US-A- 2 166 181**

(73) Patentinhaber : **Agfa-Gevaert AG**
**Kaiser-Wilhelm-Allee**
**D-51373 Leverkusen (DE)**

(72) Erfinder : **Wolff, Erich, Dr.**
**Balkhauser Weg 6**
**D-5650 Solingen (DE)**

EP 0 372 281 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft ein fotografisches Aufzeichnungsmaterial mit in diffusionsfester Form eingelagerten fotografischen "Bausteinen". Hierbei handelt es sich um fotografisch nützliche organische Verbindungen der verschiedensten Art mit sekundär-verzweigten Alkylresten. Aufgrund dieser besonderen Struktur haben die erfindungsgemäßen Bausteine niedrigen Schmelzpunkt, gute Löslichkeit in Ölbildnern und geringe Kristallisationstendenz, was sie für die fotografische Anwendung besonders geeignet macht, beispielsweise im Hinblick auf die Erzielung einer hohen maximalen Farbdichte, einer steilen Gradation einer hohen Farbausbeute (wichtig für die Ermöglichung eines geringeren Silberauftrags), insbesondere bei Verarbeitung in benzylalkoholfreien Farbentwicklern und einer besseren Stabilität gegenüber Abweichungen in den Verarbeitungsbedingungen. Aufgrund der oben erwähnten Substanzeigenschaften sind die erfindungsgemäßen Bausteine ferner geeignet für die Anwendung in Schnellverarbeitungsprozessen.

Bei der Herstellung von fotografischen Silberhalogenidemulsionsschichten und anderen hydrophilen Kolloidschichten eines fotografischen Silberhalogenidmaterials ist es oft notwendig, darin Komponenten, wie Farbkuppler, Maskierverbindungen, Weißkuppler, UV-Absorber und dergleichen zu verwenden, die in diesen hydrophilen Kolloidmedien nicht diffusionsfähig sind, so daß sie nicht wandern. Zu diesem Zweck werden diese Komponenten gewöhnlich in ihrem Molekül mit einer oder mehreren Ballastgruppen versehen, z.B. einer langkettigen, aliphatischen Gruppe, wie einer Alkylgruppe, die zwischen 5 und 20 C-Atome in gerader Linie enthält. Diese Ballastgruppe gibt dem Molekül einen hydrophoben Charakter und hält die Komponenten fest in der ursprünglichen, hydrophilen Kolloidschicht.

Die Methoden, nach denen diese nicht diffusionsfähigen Komponenten in die fotografischen, hydrophilen Kolloidschichten eingearbeitet werden, zeigen zahlreiche Probleme, und es sind viele Möglichkeiten erwogen worden, diese Probleme zu lösen.

Eine gewöhnlich angewendete Methode, um diese Komponenten diffusionsfest in hydrophile Kolloidmischungen, wie eine Gelatine-Silberhalogenidemulsion, einzuschließen, besteht darin, in das Molekül besagter Komponenten eine oder mehrere salzbildende Gruppen einzuführen, z.B. Carboxy- und/oder Sulfogruppen, so daß sie in der hydrophilen Kolloidmischung in Form ihrer löslichen alkalischen Metallsalze aufgelöst werden können.

Das Einbringen dieser nicht-diffusionsfähigen Komponenten, die salzbildende Gruppen enthalten, in wäßrige hydrophile Kolloidmischungen liefert häufig eine Anzahl von Schwierigkeiten. Tatsächlich sind viele dieser Komponenten, die Sulfo- und/oder Carboxylgruppen tragen, in der gewünschten Konzentration dieser alkalischen Lösung nicht löslich genug, während andere nach einiger Zeit in diesen Lösungen auskristallisieren oder Anlaß zur Ausflockung geben, wenn man diese Lösung mit dem wäßrigen, hydrophilen, kolloidalen Medium vermischt. Außerdem sind einige Verbindungen nur in hochalkalischen Lösungen löslich, die zur Verwendung im üblichen lichtempfindlichen Silberhalogenidmaterial zu basisch sind; wenn hochalkalische Lösungen verwendet werden, sollte die hydrophile Kolloidmischung danach wieder angesäuert werden, wobei Ausflockung auftreten kann und anorganische Salze gebildet werden. Ein anderes Problem, das die Anwendung dieser Komponenten betrifft, ist die Anwesenheit der wasserlöslichmachenden Gruppen, besonders der Sulfogruppen. Diese Gruppen haben nämlich einen merklichen Einfluß auf die Viskosität des hydrophilen, kolloidalen Mediums. Beispielsweise ist es schwierig, unter den gleichen Umständen reproduzierbare Viskositätswerte für hydrophile Kolloidmischungen zu erhalten, die derartige Komponenten enthalten; wenn man solche Zusammensetzungen lagert, verändert sich die Viskosität weiter.

Nach einer weiteren Methode, die z.B. in DE-B-1 127 714 beschrieben ist, werden wasserunlösliche Farbkuppler in niedrigsiedenden, hauptsächlich mit Wasser nicht mischbaren Lösungsmitteln wie Ethylacetat, Methylenchlorid, Chloroform usw. gelöst, worauf die gebildete Lösung in außerordentlich feinen Tröpfchen bei Anwesenheit eines Netz- oder Dispergiermittels in das hydrophile, kolloidale Medium dispergiert wird, wonach das Lösungsmittel durch Verdampfen entfernt wird und die Dispersion eines Farbkupplers zurückläßt, die in der hydrophilen Kolloidmischung verteilt ist.

Ein anderes Verfahren ist in US-A-2 322 027 beschrieben. Nach diesem Verfahren wird ein Farbkuppler in einem hochsiedenden öligen Lösungsmittel wie Tricresylphosphat und Di-n-butylphthalat gelöst, worauf die erhaltene Lösung in Form außerordentlich feiner Tröpfchen in die hydrophile Kolloidmischung dispergiert wird. Das ölige Lösungsmittel wird in der Mischung zurückgelassen.

Einer der Vorteile dieser zuletztgenannten Dispersionstechniken ist, Komponenten verwenden zu können, die keine salzbildenden Gruppen enthalten. Jedoch bringen diese Verfahren auch Schwierigkeiten. Tatsächlich sollten die Komponenten, die dazu bestimmt sind, um aus Lösungen in niedrigsiedenden bzw. hochsiedenden organischen Lösungsmitteln in fotografische, holloidale Medien eingelagert zu werden, eine genügende Löslichkeit in diesen Lösungsmitteln haben und in den kolloidalen Medien homogen verteilt sein, d.h. sowohl vor als auch nach dem Auftragen, um eine hydrophile Kolloidschicht eines fotografischen Materials zu bilden, so

daß keine Kristallisation der Komponente stattfindet.

Diese Bedingung begrenzt die Anzahl der Komponenten, die sich für solche Anwendungen eignen. Verschiedene Komponenten, die eine oder mehrere Ballastgruppen mit hydrophobem Charakter besitzen, sind in diesen organischen niedrigsiedenden oder hochsiedenden Lösungsmitteln nicht genügend löslich oder geben Anlaß zur Kristallisation der Komponente, entweder wenn man die organische Lösung in das hydrophile, kolloidale Medium dispergiert oder wenn man letzteres aufträgt, um eine Schicht eines fotografischen Materials zu bilden, oder wenn man dieses verarbeitet. Auf diese Weise wird der Dispersionsgrad merklich verringert und hat eine ungünstige Wirkung auf die sensitometrischen Eigenschaften des fotografischen Materials zur Folge.

In JP-A-62-85 242 sind diffusionsfeste fotografisch nützliche Verbindungen beschrieben, die mindestens eine Sulfogruppe enthalten und einen sekundär-verzweigten Alkylrest mit den Resten $R_1$ und $R_2$ aufweisen, die mindestens 2 C-Atome enthalten.

In US 2 166 181 sind phenolische Cyankuppler beschrieben, die in einer nicht-kuppelnden Stellung eine Alkoxygruppe enthalten. Die Verbindung in Spalte 2 Zeile 35 enthält einen α-sekundär-verzweigten Alkoxyrest mit zwei Resten $-C_7H_{15}$.

In JP-A-49-16 058 sind Pyrazolonkuppler beschrieben, die einen Acylrest enthalten, der sich von einer α-sekundärverzweigten Carbonsäure mit Resten $R_1$ und $R_2$ ableitet. $R_1$ und $R_2$ sind Alkyl- oder Alkenylreste mit 2 - 16 C-Atomen und einer Gesamtzahl von 10 - 20 C-Atomen.

In FR-A-2 078 921 sind wasserunlösliche fotografische Verbindungen mit einem β-sekundär-verzweigten Alkylrest mit Resten $R_1$ und $R_2$ beschrieben, die für verzweigtkettige Alkylreste mit 4 - 12 C-Atomen stehen.

Es wurde nun gefunden, daß fotografisch nützliche organische Verbindungen (Bausteine) der allgemeinen Struktur I sich durch niedrige Schmelzpunkte, gute Löslichkeit in den verschiedensten hochsiedenden Lösungsmitteln (Ölbildnern), geringe Kristallisationstendenz und eine ausgezeichnete Digestionsstabilität auszeichnen. Zudem können die genannten Ölbildner in ihrem Schmelzpunkt oder ihrem Lösungsvermögen für weitere Bausteine positiv beeinflußt werden, wenn sie der allgemeinen Struktur I entsprechen.

Gegenstand der Erfindung ist ein fotografisches Aufzeichnungsmaterial mit mindestens einer auf einem Schichtträger angeordneten lichtempfindlichen Silberhalogenidemulsionsschicht und gegebenenfalls weiteren nicht lichtempfindlichen Bindemittelschichten, das in einer lichtempfindlichen oder nicht lichtempfindlichen Schicht mindestens eine sulfogruppenfreie fotografisch nützliche organische Verbindung enthält, dadurch gekennzeichnet, daß die sulfogruppenfreie fotografisch nützliche Verbindung der folgenden Formel I entspricht

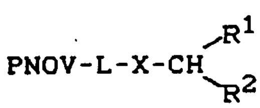

(I)

worin bedeuten

- PNOV den Rest eines Gelbkupplers oder den Rest einer fotografisch nützlichen organischen Verbindung, die keinen Bildfarbstoff bildet;
- L eine chemische Bindung oder ein Bindeglied zwischen PNOV und X;
- X -O-, -S-, -SO-, $-SO_2-$, -NH-, -NR- (R = Alkyl oder Acyl), -CO- oder Alkyliden;
- $R^1$, $R^2$ gleiche oder verschiedene unverzweigte Alkylreste mit mindestens 6 C-Atomen mit der Maßgabe, daß $R^1$ und $R^2$ zusammen mindestens 14 C-Atome und, wenn X für -CO- oder $-CH_2-$ steht, mindestens 17 C-Atome enthalten.

Ein durch L dargestelltes Bindeglied ist beispielsweise -O-, -CO-, $-SO_2-$, -NH-, -S-, Alkylen, Arylen oder eine beliebige Kombination davon. Alkylen kann geradkettig oder verzweigt sein und vorzugsweise bis zu 6 C-Atome enthalten. Arylen ist bevorzugt Phenylen, gegebenenfalls substituiert, z.B. mit Halogen, Alkyl, Alkoxy oder Acylamino.

Ein durch X dargestellter Alkylidenrest weist beispielsweise 1 bis 6 C-Atome auf; $-CH_2-$ ist ein Beispiel hierfür. Ein durch R dargestellter Alkylrest ist beispielsweise Methyl, Ethyl oder Butyl; ein durch R dargestellter Acylrest ist beispielsweise von Alkancarbonsäuren mit bis zu 6 C-Atomen abgeleitet; Acetyl ist ein Beispiel hierfür.

Bei den erfindungsgemäßen Bausteinen handelt es sich um niedermolekulare nicht-polymere Verbindungen; insbesondere sind $R^1$ und $R^2$ nicht Bestandteil einer Polymerkette.

X und L zusammen bewirken, daß das die unverzweigten Alkylreste $R^1$ und $R^2$ tragende sekundäre C-Atom (CH) von dem Rest PNOV durch mindestens ein C-Atom oder Heteroatom getrennt ist.

Bei Verbindungen, die keinen Bildfarbstoff erzeugen, kann es sich beispielsweise handeln um:

- Farbstoffe der verschiedensten Art,
- Weißkuppler,

- Entwickler, insbesondere Farbentwickler,
- Antioxidantien, Reduktoren, Elektronendonorverbindungen, Scavenger
- Ölbildner
- Beizmittel
- UV-Absorber, Weißtöner, farbstoffstabilisierende Mittel
- Komplexierungsmittel (Fixiermittel)
- Antistatika
- Deckkrafterhöhende Verbindungen
- Fungizide, Bakterizide.
- Formalinfänger
- Weichmacher.

Beispiele für erfindungsgemäße "Bausteine" sind nachfolgend aufgeführt.

Gelbkuppler

Y-1

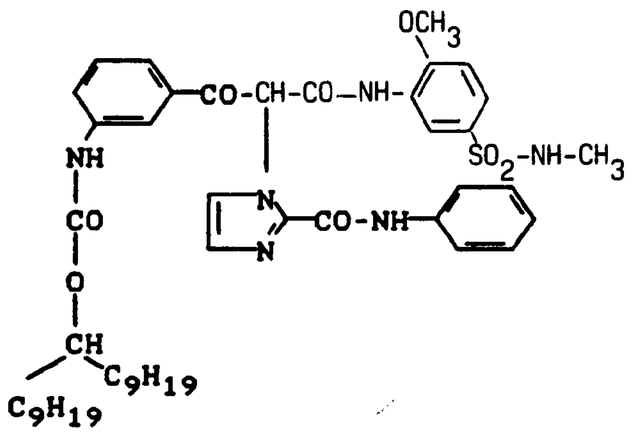

Y-2

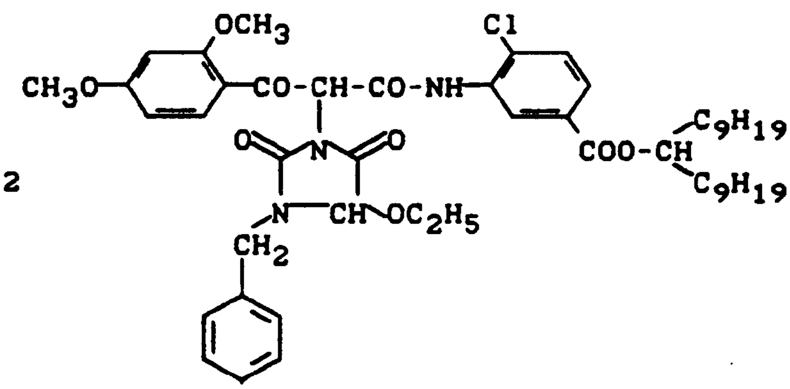

Y-3

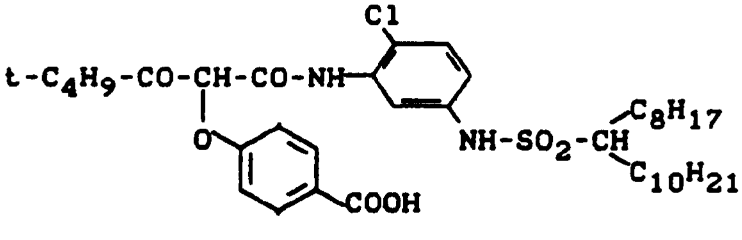

Y-4

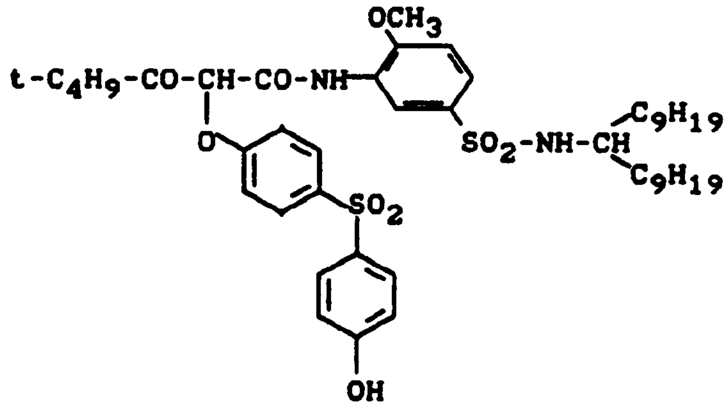

Y-5

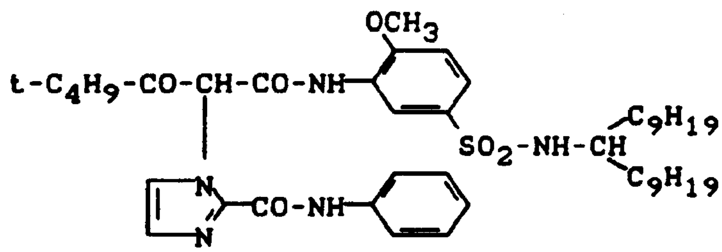

Y-6

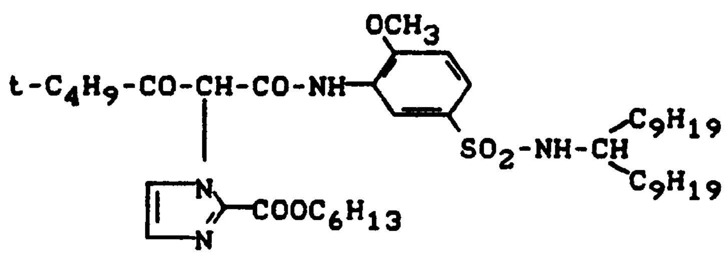

Y-7

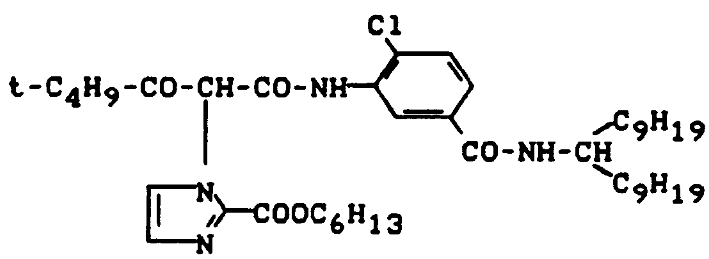

Y-8

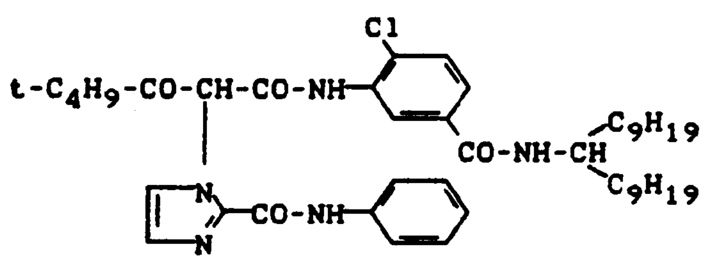

Y-9

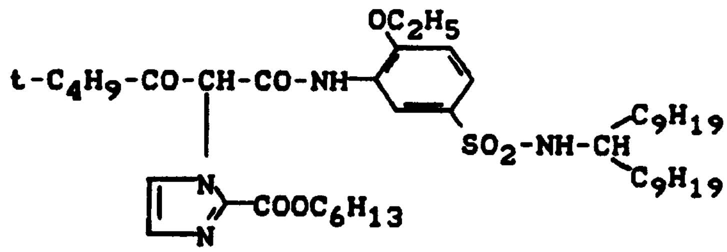

Y-10

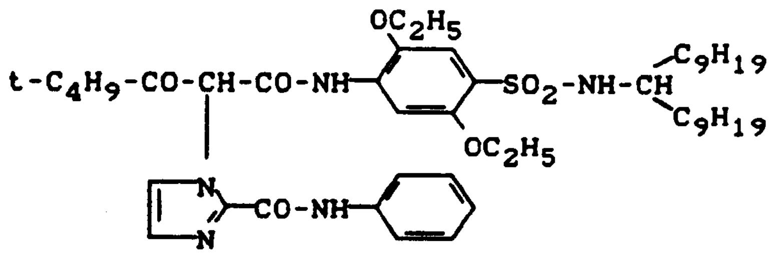

Y-11

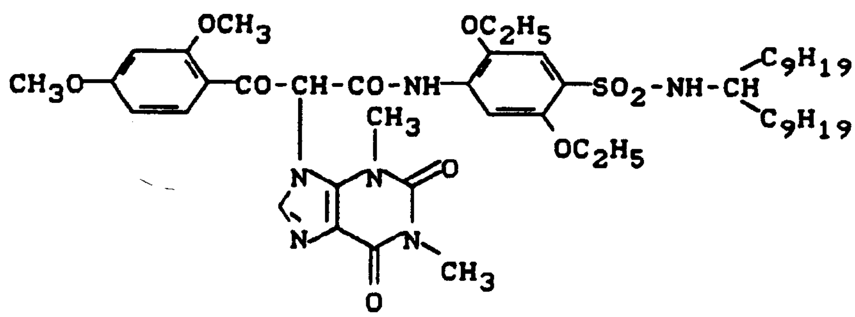

Y-12

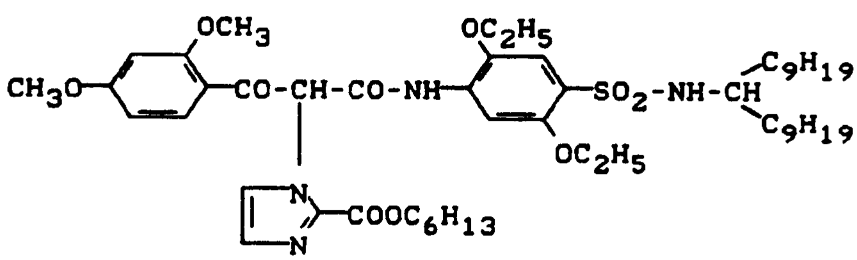

Y-13

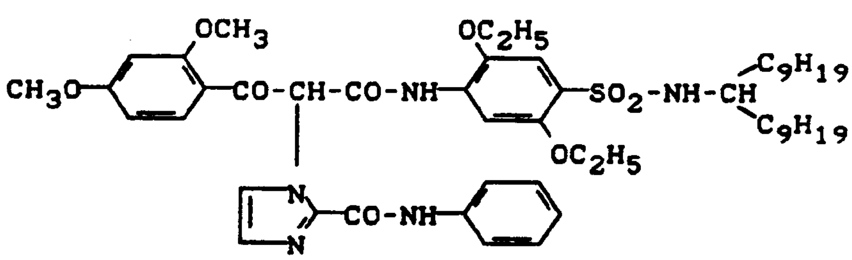

Y-14

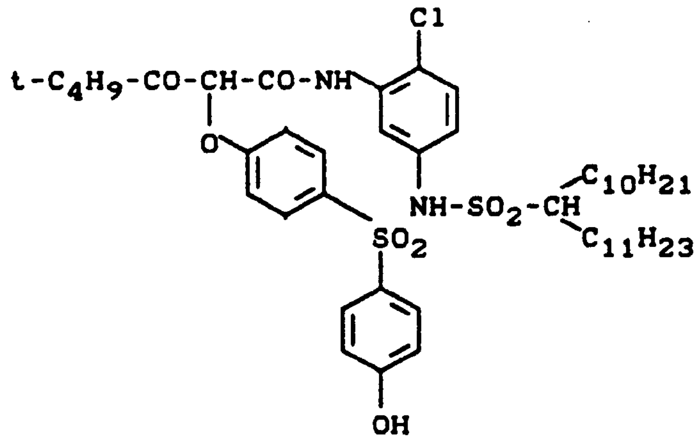

Y-15

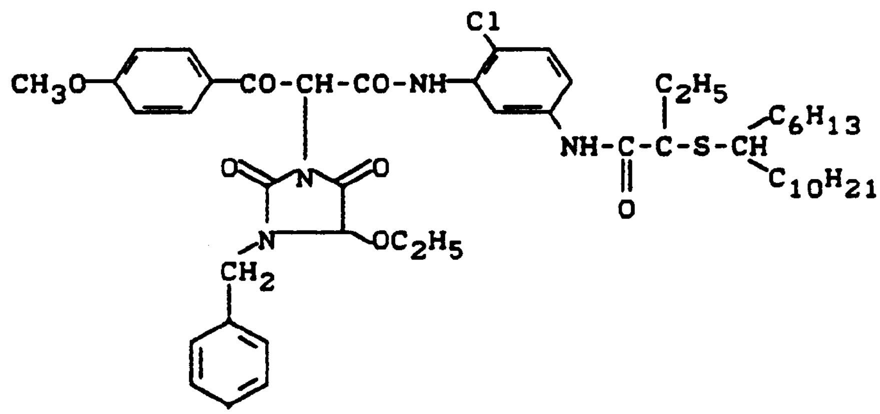

Y-16

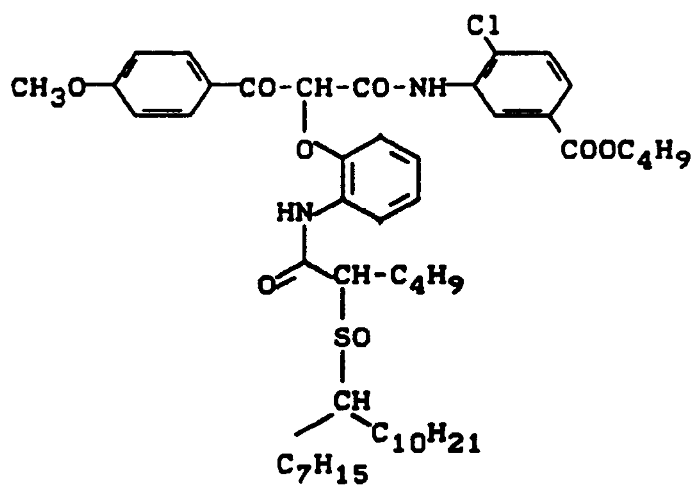

Y-17 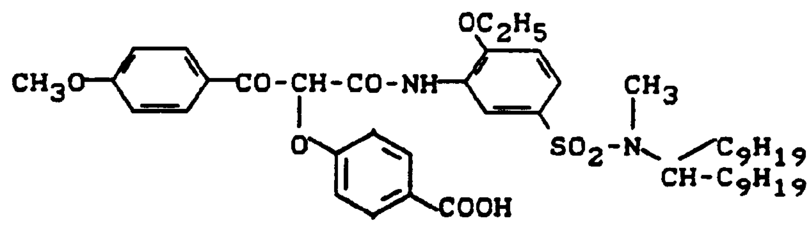

Y-18 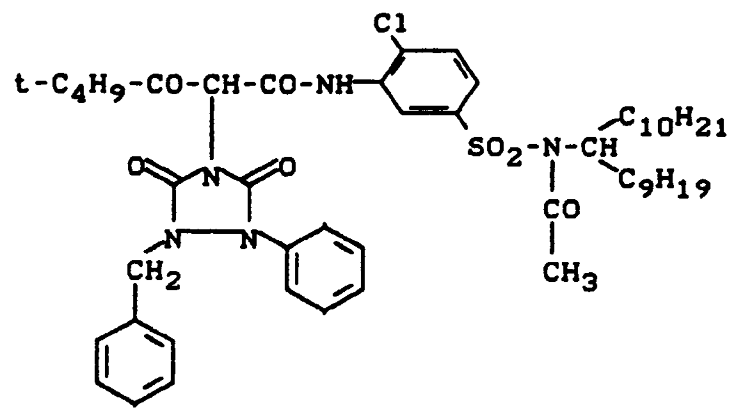

Y-19 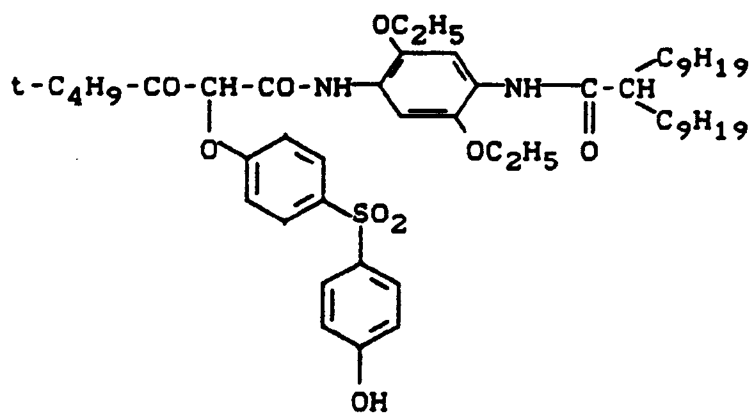

Y-20 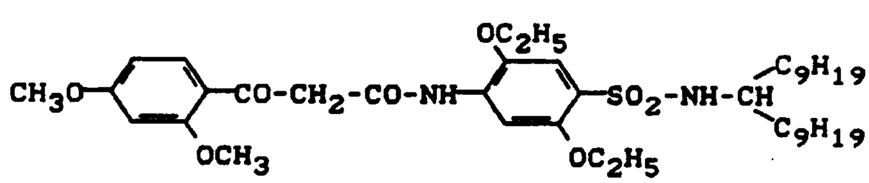

Y-21 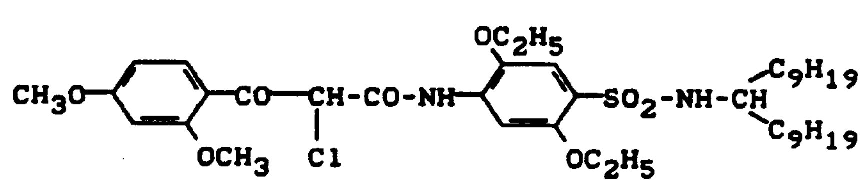

DIR-4

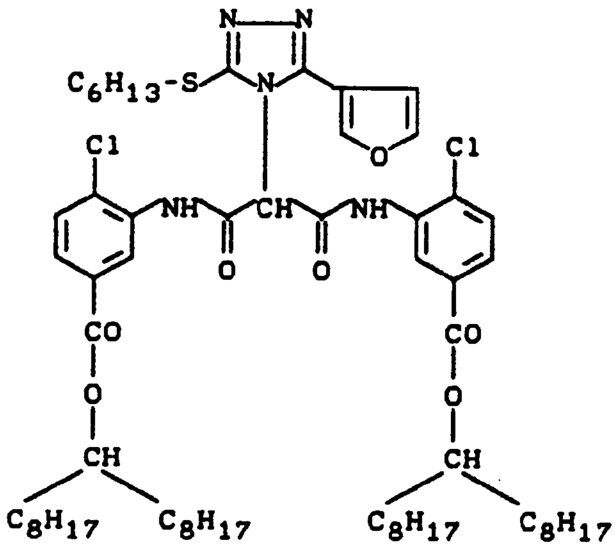

DIR-5

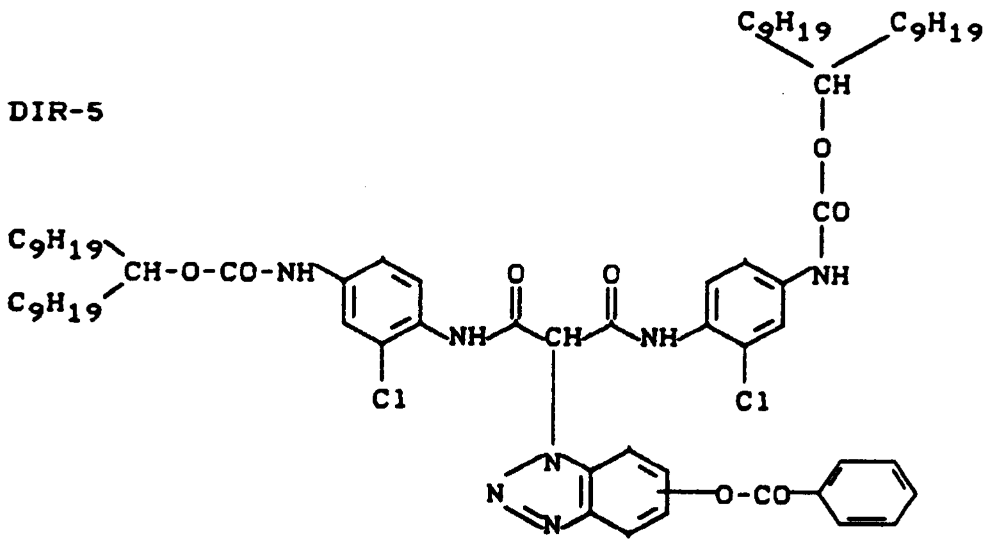

DIR-7

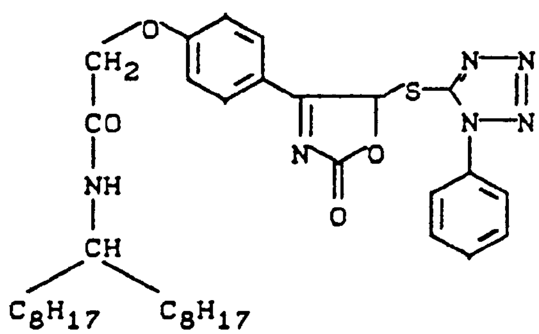

DIR-9

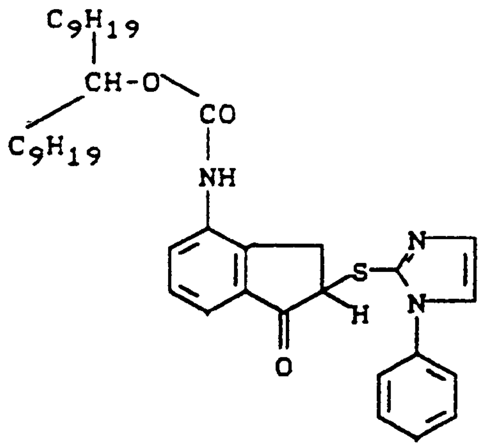

Weißkuppler

W-1

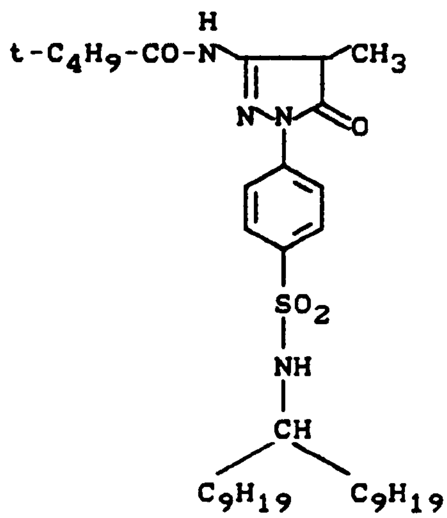

W-2

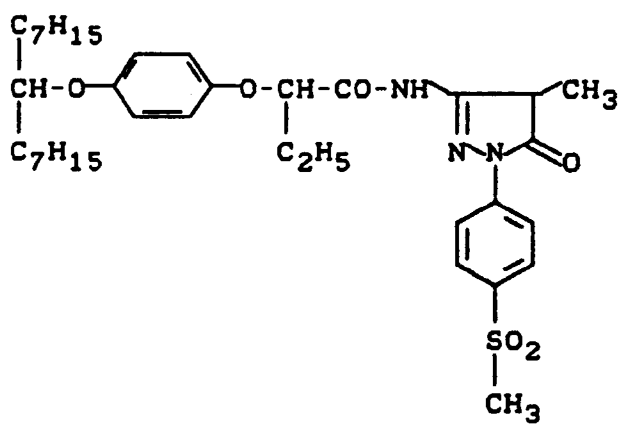

W-3

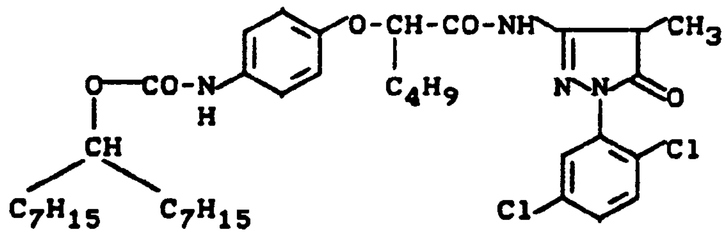

UV-Absorber

UV-1

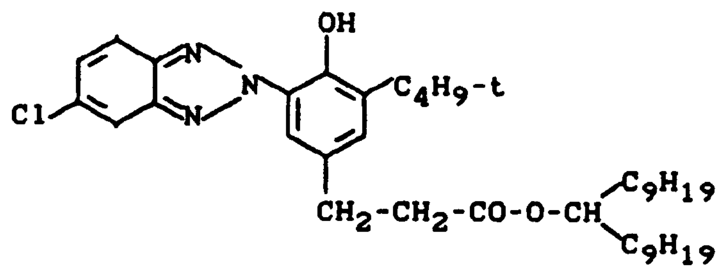

UV-2

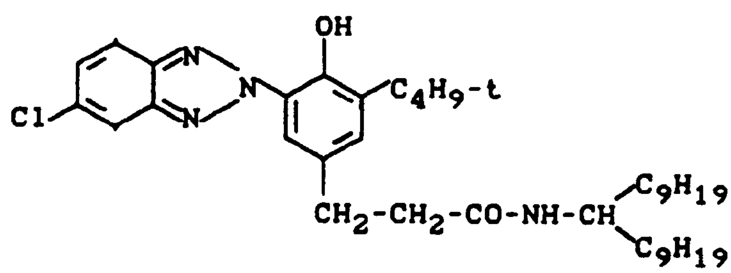

UV-3

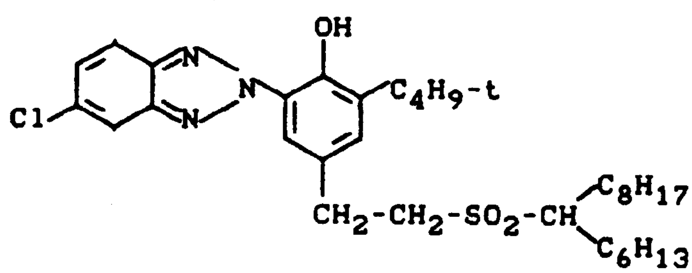

UV-4

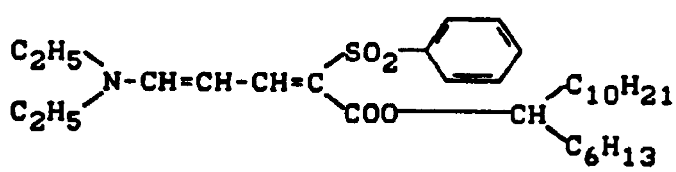

UV-5

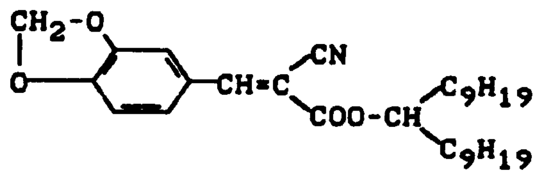

UV-6

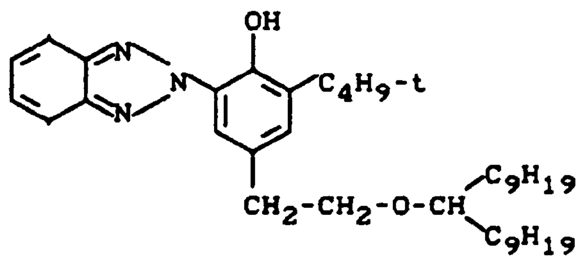

UV-7

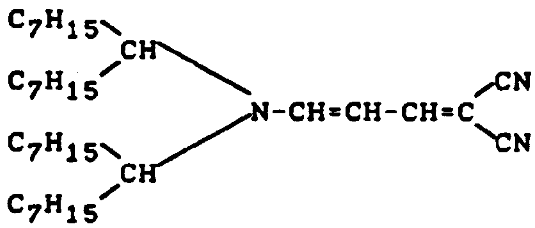

UV-8

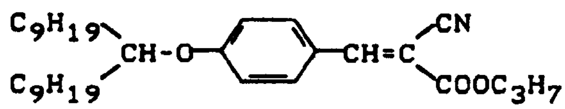

Ölbildner

OF-1

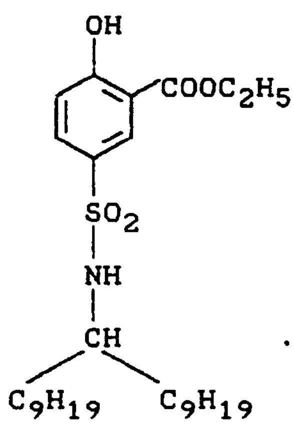

OF-2

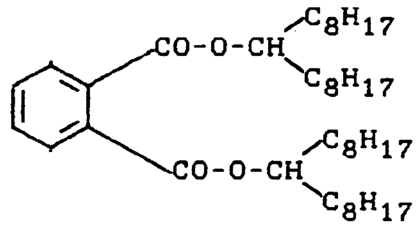

OF-3

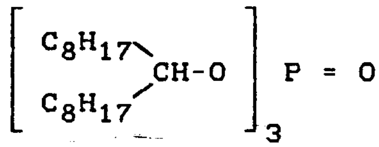

OF-4

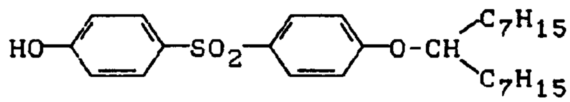

OF-5

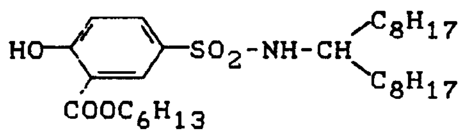

OF-6

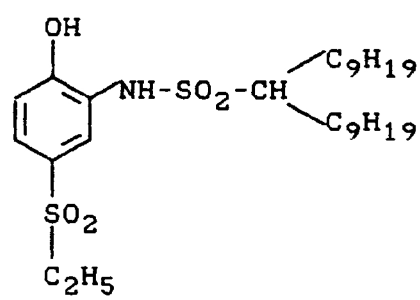

Stabilisatoren

ST-1

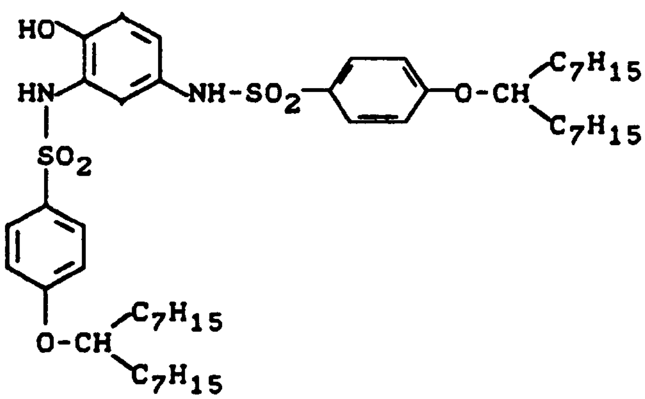

ST-2 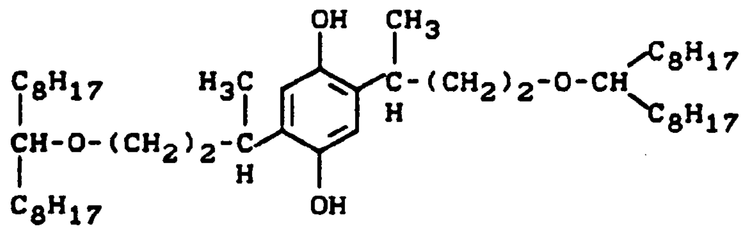

ST-3 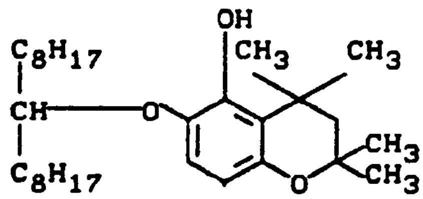

Die Synthese der erfindungsgemäßen Verbindungen erfolgt nach bekannten Verfahren. Die sekundären Alkylreste

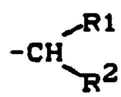

können dabei in einfacher Weise ausgehend von den entsprechenden Ketonen synthetisiert werden.

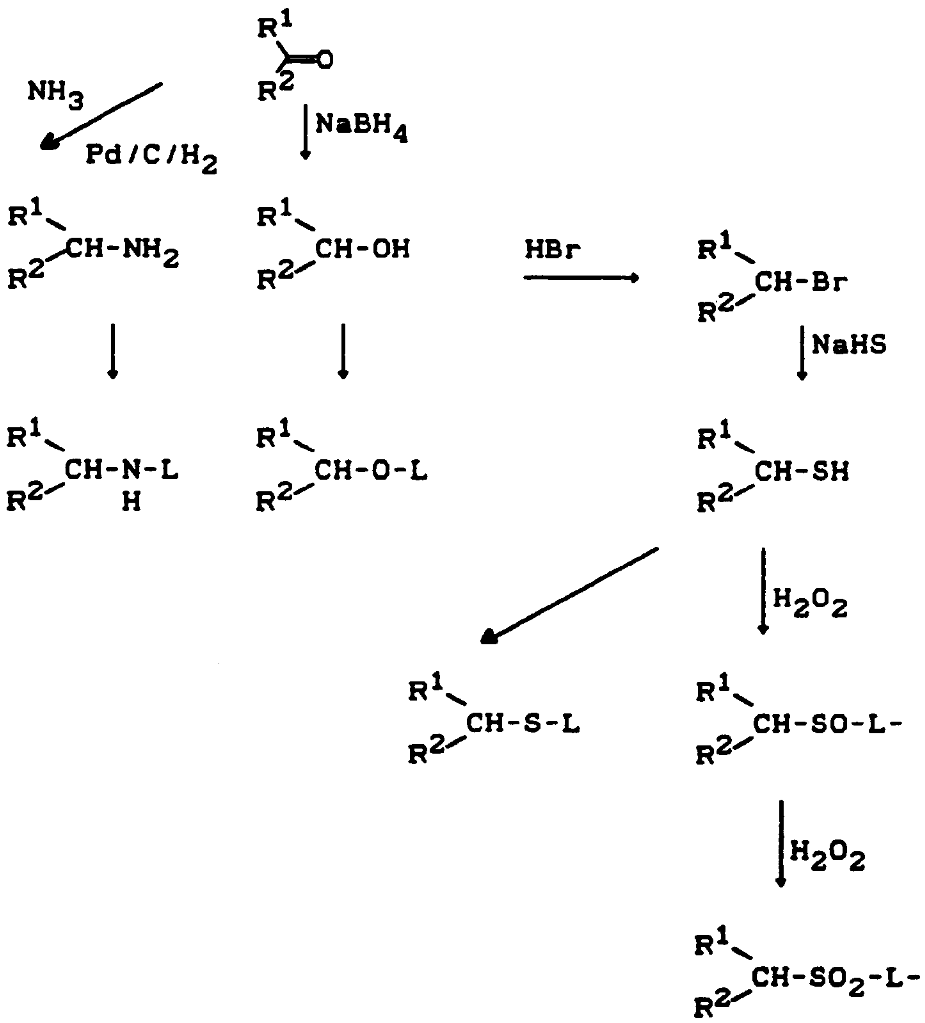


Die Zwischenprodukte sind zum Teil bereits literaturbekannt, z.B.:

| | |
|---|---|
| Nonadecan-7-ol: | Breusch, Sohullu: B. 86, [1953] 678,683 |
| Nonadecan-8-ol: | Breusch, Sohullu: B. 86, [1953] 678,682 |
| Nonadecan-9-ol: | Breusch, Sohullu: B. 86, [1953] 678,682 |
| Nonadecan-10-ol: | Carrington, Evans: Soc. 1957, 1701,1707 |
| | Meakins, Sack: Austr. J. Scient. Res. [A] 4 [1951] 213,227 |

Andere Zwischenprodukte können über die folgenden CAS-Registry-Numbers leicht recherchiert werden

| | >CH-OH | >CH-NH$_2$ | >CH-SH |
|---|---|---|---|
| 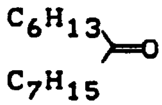 6137-34-4 | 3981-79-1 | 35601-41-3 | 95596-42-2 |
| 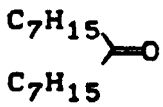 818-23-5 | 1653-35-6 | 18618-64-9 | 52911-92-9 |

| | >CH-OH | >CH-NH$_2$ | >CH-SH |
|---|---|---|---|
| 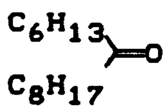 6064-38-6 | 4104-59-0 | 74856-27-2 | - |
| 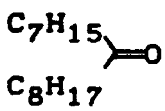 18277-02-6 | 19781-83-0 | 24539-83-1 | - |
| 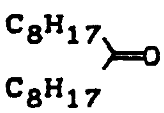 540-08-9 | 624-08-8 | 3241-20-1 | - |
| 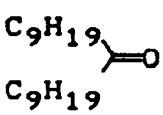 504-57-4 | 16840-84-9 | 3241-23-4 | - |

Aus symmetrischen Ketonen sind geeignete Zwischenprodukte (Hydroxyverbindungen, Amine) für erfindungsgemäße fotografisch nützliche organische Verbindungen mit $R^1 = R^2$ besonders gut zugänglich.

<u>Synthese Y-6</u>

100 g 10-Amino-nonadecan werden in einer Mischung aus
700 ml Dioxan und

| | |
|---|---|
| 200 ml | Pyridin gelöst und portionsweise mit |
| 88 g | 3-Nitro-4-methoxy-benzolsulfochlorid versetzt. Es wird 30 min auf 50°C erhitzt, in Eis/HCl ausgerührt und in Ethylacetat aufgenommen. Nach dem Abtrennen, Waschen und Trocknen der Ethylacetatphase wird das Lösungsmittel im Vakuum abgezogen. Der Rückstand wurde in |
| 250 ml | Methanol gelöst und bei 30°C mit 30 bar $H_2$ über Raney-Nickel hydriert. |
| | Ausbeute: 45 g 2-Methoxy-5[(10-nona-decylamino)sulfonyl]-anilin |
| 44 g | dieses Amins und |
| 22 g | Pivaloylessigsäureethylester (80 %ig) werden in |
| 300 ml | trockenem Toluol erhitzt. Nach 2 h Rückfluß wird ein Toluol/Ethanol-Gemisch abdestilliert und es werden |
| 5 ml | Pivaloylessigsäureethylester in |
| 200 ml | Toluol nachgesetzt. |
| | Nach vollständiger Umsetzung (Kontrolle durch Dünnschichtchromatographie) wird in Eis/HCl eingerührt und in Ethylacetat aufgenommen. Nach dem Abtrennen, Waschen, Trocknen und Abziehen des Lösungsmittels verbleiben 60 g eines farblosen Öls. |
| 60 g | dieses Öls werden in |
| 400 ml | trockenem Toluol gelöst und tropfenweise mit einer Mischung aus |
| 7 ml | Sulfurylchlorid und |
| 10 ml | Toluol versetzt. Nach vollständiger Umsetzung wird am Rotavapor eingeengt. |
| | Rückstand: 61 g Öl. |
| 30 g | dieses Öls werden in |
| 150 ml | Dimethylacetamid gelöst, mit |
| 14,5 g | Imidazol-2-carbonsäurehexylester versetzt und anschließend werden |
| 10 ml | Tetramethylguanidin zugetropft. Nach vollständiger Umsetzung (Kontrolle durch Dünnschichtchromatographie) wird in Eis/HCl eingerührt, und in Essigester aufgenommen. Nach dem Abtrennen, Waschen, Trocknen und Abdestillieren des Lösungsmittels verbleiben 31 g eines Öls. |
| | Durch Säulenchromatographie an Kieselgel mit Ethylacetat/Methylenchlorid (1:10) werden daraus 14 g hochreiner Kuppler Y-6 erhalten. |

<u>Synthese Y-12</u>

| | |
|---|---|
| 293 g | 2,5-Diethoxy-acetanilid werden in |
| 1,3 l | Methylenchlorid gelöst und tropfenweise mit |
| 383 ml | Chlorsulfonsäure versetzt. Bei einer Temperatur von 30°C wird 2 h nachgerührt, und anschließend mit Eis/HCl versetzt. Die Methylenchloridphase wird abgetrennt, gewaschen, getrocknet und am Rotavapor eingeengt. |
| 275 g | dieses Sulfochlorids werden vorgelegt und tropfenweise mit einer Lösung aus |
| 240 g | 10-Amino-nonadecan in |
| 240 ml | Methylenchlorid versetzt. Es wird bei 40°C 15 min nachgerührt und mit 20 % NaOH auf pH 8 eingestellt. Es wird 1 h nachgerührt, die organische Phase abgetrennt, getrocknet und am Rotavapor das Lösungsmittel abgezogen. |
| | Der Rückstand wird aus Acetonitril umkristallisiert. |
| | Ausbeute: 491 g. |
| 491 g | dieses Produktes werden in |
| 2,0 l | n-Propanol gelöst, zum Sieden erhitzt und tropfenweie mit |
| 237 ml | konzentrierter Salzsäure versetzt. Es wird 30 min nachgerührt und anschließend in Eis/HCl/Natriumacetat eingerührt. Das Produkt wird mit Ethylacetat extrahiert, abgetrennt, gewaschen und getrocknet. Nach dem Abziehen des Lösungsmittels verbleiben 310 g 2-Diethoxy-4-[10-(nonadecylaminosulfonyl)]-anilin |
| 133 g | 2,5-Diethoxy-4-[10-(nonadecylaminosulfonyl)]-anilin werden mit |
| 70 g | 2,4-Dimethoxy-benzoylessigsäureethylester in |
| 1,2 l | Toluol zum Sieden erhitzt. Dabei wird eine Mischung aus Toluol/Alkohol abdestilliert und laufend durch trockenes Toluol ersetzt. Nach 10 h wird im Vakuum zur Trockene eingeengt und der Rückstand aus Methanol umkristallisiert. |
| | Ausbeute: 117 g Y-20/Schmp.: 73°C |
| 117 g | Y-20 werden n |
| 900 ml | Methylenchlorid gelöst und bei Raumtemperatur tropfenweise mit |
| 13 ml | Sulfurylchlorid versetzt. Nach dem Abziehen des Lösungsmittels wird aus Methanol umkritallisiert. |

| | |
|---|---|
| | Ausbeute 96 g Y-21 Schmp.: 68-70°C |
| 30 g | Y-21 werden mit |
| 11,4 g | Imidazol-2-carbonsäurehexylester in |
| 250 ml | Dimethylacetamid vorgelegt und tropfenweise mit |
| 12 ml | Tetramethylguanidin versetzt. Es wird 4 h bei 70°C nachgerührt und anschließend in Eis/HCl gefällt. Es wird mit Ethylacetat extrahiert, getrennt, mit Wasser gewaschen und getrocknet und das Lösungsmittel am Rotavapor abgezogen. Rückstand: 30 g öliger Kuppler Y-12. |
| | Der gb-Kuppler Y-12 kann durch Säulenchromatographie an Kieselgel mit Ethylacetat/Methylenchlorid (1:20) als Laufmittel hochrein erhalten werden. Der Kuppler Y-12 ist nach wie vor bei Raumtemperatur nicht kristallin. |

Synthese Y-13

Aus Y-21 wird in analoger Weise mit Imidazol-2-carbonsäureanilid der Kuppler Y-13 erhalten. Y-13 ist nach säulenchromatographischer Reinigung an Kieselgel mit Ethylacetat/Methylenchlorid (1:20) ebenfalls hochrein. Er wird als halbfeste nichtkristalline Masse isoliert.

Die erfindungsgemäßen Bausteine können in fotografischen Materialien aller Art verwendet werden, insbesondere solchen, die lichtempfindliches Silberhalogenid enthalten. Es kann sich hierbei um Matrialien für die Schwarzweißfotografie oder um farbfotografische Materialien handeln.

Beispiele für farbfotografische Materialien sind Farbnegativfilme, Farbumkehrfilme, Farbpositivfilme, farbfotografisches Papier, farbumkehrfotografisches Papier, farbempfindliche Materialien für das Farbdiffusionstransfer-Verfahren oder das Silberfarbbleich-Verfahren.

Geeignete Träger zur Herstellung fotografischer Materialien sind z.B. Filme und Folien von halbsynthetischen und synthetischen Polymeren, wie Cellulosenitrat, Celluloseacetat, Cellulosebutyrat, Polystyrol, Polyvinylchlorid, Polyethylenterephthalat und Polycarbonat und mit einer Barytschicht oder α-Olefinpolymerschicht (z.B. Polyethylen) laminiertes Papier. Diese Träger können mit Farbstoffen und Pigmenten, beispielsweise Titandioxid, gefärbt sein. Sie können auch zum Zwecke der Abschirmung von Licht schwarz gefärbt sein. Die Oberfläche des Trägers wird im allgemeinen einer Behandlung unterzogen, um die Adhäsion der fotografischen Emulsionsschicht zu verbessern, beispielsweise einer Corona-Entladung mit nachfolgendem Antrag einer Substratschicht.

Die farbfotografischen Materialien enthalten üblicherweise mindestens je eine rotempfindliche, grünempfindliche und blauempfindliche Silberhalogenidemulsionsschicht mit jeweils spektral zugeordneten Farbkupplern sowie gegebenenfalls Zwischenschichten und Schutzschichten.

Als Bindemittel wird vorzugsweise Gelatine verwendet. Diese kann jedoch ganz oder teilweise durch andere synthetische, halbsynthetische oder auch natürlich vorkommende Polymere ersetzt werden. Synthetische Gelatineersatzstoffe sind beispielsweise Polyvinylalkohol, Poly-N-vinylpyrrolidon, Polyacrylamide, Polyacrylsäure und deren Derivate, insbesondere deren Mischpolymerisate. Natürlich vorkommende Gelatineersatzstoffe sind beispielsweise andere Proteine wie Albumin oder Casein, Cellulose, Zucker, Stärke oder Alginate. Halbsynthetische Gelatineersatzstoffe sind in der Regel modifizierte Naturprodukte. Cellulosederivate wie Hydroxyalkylcellulose, Carboxymethylcellulose und Phthalylcellulose sowie Gelatinederivate, die durch Umsetzung mit Alkylierungs- oder Acylierungsmitteln oder durch Aufpfropfung von polymerisierbaren Monomeren erhalten worden sind, sind Beispiele hierfür.

Die Bindemittel sollen über eine ausreichende Menge an funktionellen Gruppen verfügen, so daß durch Umsetzung mit geeigneten Härtungsmitteln genügend widerstandsfähige Schichten erzeugt werden können. Solche funktionellen Gruppen sind insbesondere Aminogruppen, aber auch Carboxylgruppen, Hydroxylgruppen und aktive Methylengruppen.

Die vorzugsweise verwendete Gelatine kann durch sauren oder alkalischen Aufschluß erhalten worden sein. Es kann auch oxidierte Gelatine verwendet werden. Die Herstellung solcher Gelatinen wird beispielsweise in The Science and Technology of Gelatine, herausgegeben von A.G. Ward und A. Courts, Academic Press 1977, Seite 295 ff beschrieben. Die jeweils eingesetzte Gelatine soll einen möglichst geringen Gehalt an fotografisch aktiven Verunreinigungen enthalten (Inertgelatine). Gelatinen mit hoher Viskosität und niedriger Quellung sind besonders vorteilhaft.

Das als lichtempfindlicher Bestandteil in dem fotografischen Material befindliche Silberhalogenid kann als Halogenid Chlorid, Bromid oder Iodid bzw. Mischungen davon enthalten. Beispielsweise kann der Halogenidanteil wenigstens einer Schicht zu 0 bis 15 mol-% aus Iodid, zu 0 bis 100 mol-% aus Chlorid und zu 0 bis 100 mol-% aus Bromid bestehen. Im Falle von Farbnegativ- und Farbumkehrfilmen werden üblicherweise Silberbromidiodidemulsionen, im Falle von Farbnegativ- und Farbumkehrpapier üblicherweise Silberbromidemulsionen oder Silberchloridbromidemulsionen mit hohem Chloridanteil bis zu reinen Silberchloridemulsionen verwendet. Es kann sich um überwiegend kompakte Kristalle handeln, die z.B. regulär kubisch oder oktaedrisch sind oder Übergangsformen aufweisen können. Vorzugsweise können aber auch plättchenförmige Kristalle vorliegen, deren durchschnittliches Verhältnis von Durchmesser zu Dicke bevorzugt wenigstens 5:1 ist, wobei der Durchmesser eines Kornes definiert ist als der Durchmesser eines Kreises mit einem Kreisinhalt entsprechend der projizierten Fläche des Kornes. Die Schichten können aber auch tafelförmige Silberhalogenidkristalle aufweisen, bei denen das Verhältnis von Durchmesser zu Dicke wesentlich größer als 5:1 ist, z.B. 12:1 bis 30:1.

Die Silberhalogenidkörner können auch einen mehrfach geschichteten Kornaufbau aufweisen, im einfachsten Fall mit einem inneren und einem äußeren Kornbereich (core/shell), wobei die Halogenidzusammensetzung und/oder sonstige Modifizierungen, wie z.B. Dotierungen der einzelnen Kornbereiche unterschiedlich sind. Die mittlere Korngröße der Emulsionen liegt vorzugsweise zwischen 0,2 µm und 2,0 µm, die Korngrößenverteilung kann sowohl homo- als auch heterodispers sein. Homodisperse Korngrößenverteilung bedeutet, daß 95 % der Körner nicht mehr als ± 30% von der mittleren Korngröße abweichen. Die Emulsionen können neben dem Silberhalogenid auch organische Silbersalze enthalten, z.B. Silberbenztriazolat oder Silberbehenat.

Die Silberhalogenidemulsion wird im allgemeinen einer chemischen Sensibilisierung unter definierten Bedingungen - pH, pAg, Temperatur, Gelatine-, Silberhalogenid- und Sensibilisatorkonzentration - bis zum Erreichen des Empfindlichkeits- und Schleieroptimums unterworfen. Die Verfahrensweise ist z.B. bei H. Frieser "Die Grundlagen der Photographischen Prozesse mit Silberhalogeniden" Seite 675-734, Akademische Verlagsgesellschaft (1968) beschrieben.

Die fotografischen Emulsionen können Verbindungen zur Verhinderung der Schleierbildung oder zur Stabilisierung der fotografischen Funktion während der Produktion, der Lagerung oder der fotografischen Verarbeitung enthalten.

Die fotografischen Emulsionen können unter Verwendung von Methinfarbstoffen oder anderen Farbstoffen spektral sensibilisiert werden. Besonders geeignete Farbstoffe sind Cyaninfarbstoffe, Merocyaninfarbstoffe und komplexe Merocyaninfarbstoffe.

Eine Übersicht über die als Spektralsensibilisatoren geeigneten Polymethinfarbstoffe, deren geeignete Kombinationen und supersensibilisierend wirkenden Kombinationen enthält Research Disclosure 17643/1978 in Abteilung IV.

Auf Sensibilisatoren kann verzichtet werden, wenn für einen bestimmten Spektralbereich die Eigenempfindlichkeit des Silberhalogenids ausreichend ist, beispielsweise die Blauempfindlichkeit von Silberbromiden.

Den unterschiedlich sensibilisierten Emulsionsschichten werden nicht diffundierende monomere oder polymere Farbkuppler zugeordnet, die sich in der gleichen Schicht oder in einer dazu benachbarten Schicht befinden können. Gewöhnlich werden den rotempfindlichen Schichten Blaugrünkuppler, den grünempfindlichen Schichten Purpurkuppler und den blauempfindlichen Schichten Gelbkuppler zugeordnet.

Die Einarbeitung der Kuppler oder anderer Verbindungen in Silberhalogindemulsionsschichten kann in der Weise erfolgen, daß zunächst von der betreffenden Verbindung eine Lösung, eine Dispersion oder eine Emulsion hergestellt und dann der Gießlösung für die betreffende Schicht zugefügt wird. Die Auswahl des geeigneten Lösungs- oder Dispersionsmittel hängt von der jeweiligen Löslichkeit der Verbindung ab. Die erfindungsgemäßen Bausteine können wegen ihrer hervorragenden Löslichkeit als Emulsion verarbeitet und in die Schichten eingebracht werden.

Hydrophobe Verbindungen können auch unter Verwendung von hochsiedenden Lösungsmitteln, sogenannten Ölbildnern, in die Gießlösung eingebracht werden. Entsprechende Methoden sind beispielsweise in US-A-2 322 027, US-A-2 801 170, US-A-2 801 171 und EP-A-O 043 037 beschrieben.

Anstelle der hochsiedenden Lösungsmitteln können Oligomere oder Polymere, sogenannte polymere Ölbildner Verwendung finden.

Die Verbindungen können auch in Form beladener Latices in die Gießlösung eingebracht werden. Verwiesen wird beispielsweise auf DE-A-25 41 230, DE-A-25 41 274, DE-A-28 35 856, EP-A-0 014 921, EP-A-0 069 671, EP-A-0 130 115, US-A-4 291 113.

Geeignete Ölbildner sind z.B. Phthalsäurealkylester, Phosphonsäureester, Phosphorsäureester, Citronensäureester, Benzoesäureester, Amide, Fettsäureester, Trimesinsäureester, Alkohole, Phenole, Anilinderivate und Kohlenwasserstoffe.

Beispiele für geeignete Ölbildner sind Dibutylphthalat, Dicyclohexylphthalat, Di-2-ethylhexylphthalat, Decylphthalat, Triphenylphosphat, Tricresylphosphat, 2-Ethylhexyldiphenylphosphat, Tricyclohexylphosphat, Tri-2-ethylhexylphosphat, Tridecylphosphat, Tributoxyethylphosphat, Trichlorpropylphosphat, Di-2-ethylhexylphenylphosphat, 2-Ethylhexylbenzoat, Dodecylbenzoat, 2-Ethylhexyl-p-hydroxybenzoat, Diethyldodecanamid, N-Tetradecylpyrrolidon, Isostearylalkohol, 2,4-Di-tert.-amylphenol, Dioctylacelat, Glycerintributyrat, Isostearyllactat, Trioctylcitrat, N,N-Dibutyl-2-butoxy-5-tert.-octylanilin, Paraffin, Dodecylbenzol und Diisopropylnaphthalin. Anstelle der genannten Ölbildner können auch solche der Formel I mit Vorteil verwendet werden.

Beispiel 1

Herstellung und Beurteilung der Kuppleremulgate Jeweils 8 mmol Kuppler wurden im Verhältnis 1:3 in ca. 50°C warmen Ethylacetat (EA) gelöst und mit Dibutylphthalat (DBP) sowie Manoxol versetzt, so daß ein Verhältnis:

Kuppler : DBP : EA : Manoxol = 1:1:3:0,1

resultiert. Anschließend wurde in 7,5 %iger Gelatinelösung emulgiert. Abhängig vom Molgewicht ergibt sich ein Verhältnis Kuppler:Gelatine von ca. 1:2. Das Emulgat wurde 6 min bei 1000 u/min gerührt, wobei es sich auf ca. 50°C erwärmte und wobei EA im Wasserstrahlvakuum (200-300 mbar) abdestilliert wurde.

Die Qualität der frischen Kuppleremulgate wurde mit Hilfe eines Phasenkontrast- bzw. Polarisationsmikroskops folgendermaßen bewertet:

a) Teilchengröße
- 1 = sehr fein (<0,5 µm)
- 2 = fein (< 1 µm)
- 3 = fein mit einigen größeren Teilchen
- 4 = mittel
- 5= grob

b) Homogenität
- 1 = keine Kristalle erkennbar
- 2 = vereinzelt Kristalle erkennbar
- 3 = viele Kristalle erkennbar
- 4 = stark auskristallisiert

Die gleiche Beurteilung erfolgte, nachdem die Emulgate 3 h bzw. 6 h bei 50°C intensiv gerührt worden waren.

Beispiel 2

Vergleich verschiedener Gelbkuppler

| | | Fp. |
|---|---|---|
| Y-6 | | Öl |
| Y-10 | | Öl |
| Y-12 | | Öl |
| Y-13 | | halbfest |
| VY-1 | 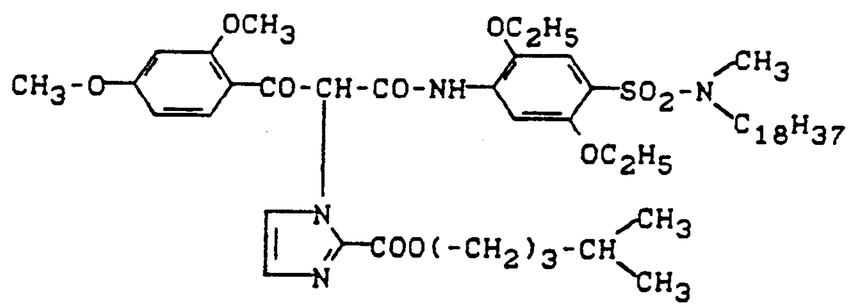 | 67° C |
| VY-2 | 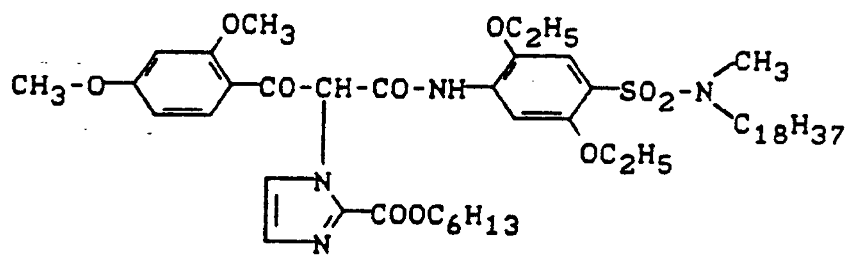 | 57° C |

Bereits beim Vergleich der Schmelzpunkte ist der Vorteil der erfindungsgemäßen Verbindungen deutlich erkennbar.

Darüber hinaus zeichnen sich die Verbindungen Y-6, Y-10, Y-12, Y-13 durch eine deutlich bessere Löslichkeit in Trikresylphosphat und Dibutylphthalat aus.

Die genannten Verbindungen wurden gemäß Beispiel 1 emulgiert und die erhaltenen Kuppleremulgate wie in Beispiel 1 visuell bewertet.

**Tabelle 2**

| | Qualität der Kuppleremulgate | | | | | |
|---|---|---|---|---|---|---|
| | frisch | | nach 3 h/50° C | | nach 6 H/50° C | |
| | a | b | a | b | a | b |
| Y-6 | 1 | 1 | 1 | 1 | 1 | 2 |
| Y-10 | 1 | 2 | 1 | 2 | 1 | 2 |
| Y-12 | 1 | 1 | 1 | 1 | 1 | 1 |
| Y-13 | 1 | 1 | 1 | 2 | 1 | 1 |
| VY-1 | 3 | 3 | 3 | 3 | 3 | 4 |
| VY-2 | 4 | 3 | 4 | 4 | 4 | 4 |

Die gemäß Beispiel 1 hergestellten Emulgate wurden mit einer Silberbromidiodidemulsion (0,7 mol-% Iodid) im Verhältnis 1 mol Kuppler:5,2 mol $AgNO_3$ abgemischt, auf einen Schichtträger aus Celluloseacetat aufgetragen und mit einer Schutzschicht aus einer 3 %igen Gelatinelösung überschichtet, die als Härtungsmittel ein Cabamoylpyridiniumbetain (CAS Reg. No 65411-60-1) enthielt. Nach dem Trocknen und Aufschneiden wurden die so hergestellten Proben hinter einem Stufenkeil belichtet und wie folgt einmal mit und einmal ohne Benzylalkohol verarbeitet (30°C):

| | |
|---|---|
| Entwickeln | 210 s |
| Bleichfixieren | 90 s |
| Wässern | 120 s |

Die Bäder hatten folgende Zusammensetzung:

| | |
|---|---|
| 5,0 g | 4-Amino-3-methyl-N-ethyl-N-(β-methansulfonamidoethyl)-anilin-sulfat |
| (15,0 ml | Benzylalkohol) |
| 2,5 g | Natriumhexametaphosphat |
| 1,85 g | $Na_2SO_3$ sicc. |
| 1,4 g | NaBr |
| 0,5 g | KBr |
| 39,1 g | Borax |

Auffüllen mit Wasser auf 1000 ml; mit NaOH auf pH 10,3 einstellen.

Bleichfixierbad:

| | |
|---|---|
| 50,0 g | Ethylendiamintetraessigsäure-Eisen(III)-Ammonium-Komplex |
| 50,0 ml | $(NH_4)_2SO_3$, 40 %ige Lösung |
| 140,0 ml | $(NH_4)_2SSO_3$, 70 %ige Lösung |
| 20,0 ml | wäßriger Ammoniak, 28 %ig |
| 4,0 g | Ethylendiamintetraessigsäure |

Auffüllen mit Wasser auf 1000 ml.

Für jede der hergestellten Proben sind in der Tabelle 3 die relative Empfindlichkeit und die maximale Farbdichte angegeben. Für die Empfindlichkeit werden relative Werte angegeben, bezogen auf die höchste erzielte Empfindlichkeit (= 100). Die bei Entwicklung in Gegenwart von Benzylalkohol erzielten Werte sind in Klammern aufgeführt.

**Tabelle 3**

| | rel. Empfindlichkeit | | $D_{max}$ | | FA ohne Benzylalkohol |
|---|---|---|---|---|---|
| VY-1 | 85 | (94) | 1,5 | (2,0) | 1,0 |
| VY-2 | 87 | (93) | 1,6 | (2,1) | 1,06 |
| Y-6 | 96 | (97) | 2,5 | (2,5) | 1,66 |
| Y-10 | 99 | (100) | 2,6 | (2,7) | 1,73 |
| Y-12 | 98 | (98) | 2,8 | (2,8) | 1,86 |
| Y-13 | 98 | (98) | 2,4 | (2,4) | 1,60 |

Die Kuppler gemäß der Erfindung zeichnen sich in Entwicklern mit Benzylalkohol durch eine deutlich höhere maximale Dichte aus; vor allen Dingen aber bleibt diese höhere Dichte auch in benzylalkoholfreien Entwicklern erhalten. Dies ist für neue Verarbeitungsprozesse in Zukunft aus ökologischer Sicht von besonderer Bedeutung. Aufgrund der verbesserten Farbausbeute FA wird außerdem eine beträchtliche Einsparung an Silber möglich.

**Patentansprüche**

1. Fotografisches Aufzeichnungsmaterial mit mindestens einer auf einem Schichtträger angeordneten lichtempfindlichen Silberhalogenidemulsionsschicht und gegebenenfalls weiteren, nicht lichtempfindlichen

Bindemittelschichten, das in einer lichtempfindlichen oder nicht lichtempfindlichen Schicht mindestens eine sulfogruppenfreie fotografisch nützliche organische Verbindung enthält, dadurch gekennzeichnet, daß die sulfogruppenfreie fotografisch nützliche organische Verbindung der folgenden Formel entspricht:

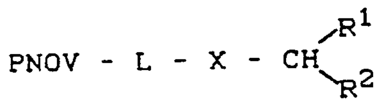

worin bedeuten

PNOV den Rest eines Gelbkupplers oder den Rest einer fotografisch nützlichen organischen Verbindung, die keinen Bildfarbstoff bildet;

L ein Bindeglied zwischen PNOV und X;

X -O-, -S-, -SO-, $-SO_2-$, -NH-, -NR- (R = Alkyl oder Acyl), -CO- oder Alkyliden;

$R^1$, $R^2$ unverzweigte Alkylreste mit mindestens 6 C-Atomen, mit der Maßgabe, daß $R^1$ und $R^2$ zusammen mindestens 14 C-Atome und wenn X für -CO- oder $-CH_2-$ steht, mindestens 17 C-Atome enthalten.

2. Aufzeichnungsmaterial nach Anspruch 1, dadurch gekennzeichnet, daß in der allgemeinen Formel des Anspruchs 1 L für -O-, -CO-, $-SO_2-$, -NH-, -S-, Alkylen, Arylen oder eine beliebige Kombination davon steht.

3. Aufzeichnungsmaterial nach Anspruch 1, dadurch gekennzeichnet, daß die fotografisch nützliche organische Verbindung ein Ölbildner ist.

## Claims

1. A photographic recording material which comprises at least one photosensitive silver halide emulsion layer arranged on a layer support and, optionally, other non-photosensitive binder layers and which, in a photosensitive or non-photosensitive layer, contains at least one photographically useful organic compound free from sulfo groups, characterized in that the photographically useful organic compound free from sulfo groups corresponds to the following formula:

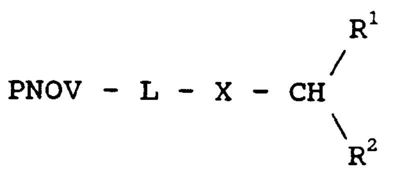

(I)

in which

PNOV is the residue of a yellow coupler or the residue of a photographically useful organic compound that does not form an image dye;

L is a binding link between PNOV and X;

X represents -O-, -S-, -SO-, $-SO_2-$, -NH-, -NR- (R = alkyl or acyl), -CO- or alkylidene;

$R^1$ and $R^2$ are unbranched alkyl radicals containing at least 6 C atoms, with the proviso that $R^1$ and $R^2$ together contain at least 14 C atoms and, where X represents -CO- or $-CH_2$, at least 17 C atoms.

2. A recording material as claimed in claim 1, characterized in that, in the general formula in claim 1, L represents -O-, -CO-, $-SO_2-$, -NH-, -S-, alkylene, arylene or any combination thereof.

3. A recording material as claimed in claim 1 or 2, characterized in that the photographically useful organic compound is an oil former.

## Revendications

1. Matériau de reproduction photographique avec au moins une couche d'émulsion d'halogénure d'argent photosensible disposée sur un support de couches et éventuellement d'autres couches de liants non photosensibles, qui contient dans une couche photosensible ou non au moins un composé organique dépourvu de radical sulfo, photographiquement utile, caractérisé en ce que le composé organique dépourvu de radical sulfo photographiquement utile correspond à la formule suivante:

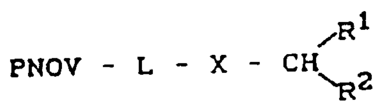

dans laquelle

PNOV désigne le radical d'un coupleur en jaune ou le radical d'un composé organique photographiquement utile qui ne forme pas de colorant de l'image;

L un élément de liaison entre PNOV et X;

X désigne -O-, -S-, -SO-, $-SO_2-$, -NH-, -NR- (R = alkyle ou acyle), -CO- ou des alkylidènes;

$R^1$, $R^2$ désignent des résidus alkyle non ramifiés, identiques ou différents, comportant au moins 6 atomes de C dans la mesure où $R^1$ et $R^2$ contiennent ensemble au moins 14 atomes de C et, si X remplace -CO- ou $-CH_2-$, au moins 17 atomes de C.

2. Matériau de reproduction photographique selon la revendication 1, caractérisé en ce que dans la formule générale de la revendication 1, L désigne -O-, -CO-, $-SO_2-$, -NH-, -S-, un alkylène, un arylène ou une combinaison quelconque de ces éléments.

3. Matériau de reproduction photographique selon la revendication 1, caractérisé en ce que le composé organique photographiquement utile est un agent oléifiant.